(19) **Europäisches Patentamt**

**European Patent Office**

**Office européen des brevets**

(11) Veröffentlichungsnummer: **0 238 701 B1**

(12) ## EUROPÄISCHE PATENTSCHRIFT

(45) Veröffentlichungstag der Patentschrift: **18.03.92**

(51) Int. Cl.⁵: **C07K 3/24**, A61K 35/16

(21) Anmeldenummer: **86104297.6**

(22) Anmeldetag: **27.03.86**

Die Akte enthält technische Angaben, die nach dem Eingang der Anmeldung eingereicht wurden und die nicht in dieser Patentschrift enthalten sind.

(54) **Verfahren zur Herstellung eines hochgereinigten Antihämophilie-Faktors.**

(43) Veröffentlichungstag der Anmeldung:
**30.09.87 Patentblatt 87/40**

(45) Bekanntmachung des Hinweises auf die Patenterteilung:
**18.03.92 Patentblatt 92/12**

(84) Benannte Vertragsstaaten:
**AT DE FR GB IT NL SE**

(56) Entgegenhaltungen:
**AT-A- 349 639**      **FR-A- 2 184 898**
**FR-A- 2 411 608**      **US-A- 4 170 639**
**US-A- 4 478 825**      **US-A- 4 543 210**

(73) Patentinhaber: **Octapharma AG**
**Bankstrasse 7**
**CH-8750 Glarus(CH)**

(72) Erfinder: **Smith, Andrew**
**Bankstrasse 7**
**CH-8750 Glarus(CH)**

(74) Vertreter: **Werner, Hans-Karsten, Dr. et al**
**Deichmannhaus am Hauptbahnhof**
**W-5000 Köln 1(DE)**

## Beschreibung

Gegenstand der vorliegenden Erfindung ist ein Verfahren zur Herstellung eines hochgereinigten Antihämophilie-Faktors (AHF oder Faktor VIII) durch Abtrennung von Fibrinogen, Globulin und Albumin aus dem Kryoprezipitat.

Es ist bekannt, daß das Kryoprezipitat nach Cohn überwiegend aus Fibrinogen, Globulin und Albumin besteht und darüber hinaus die Hauptmenge des Antihämophilie-Faktors oder Faktors VIII nach Cohn. Weiterhin enthält das Kryoprezipitat noch gewisse Mengen der Blutfaktoren VII, IX, X und Prothrombin, jedoch sind diese Verunreinigungen relativ gering gegenüber den Hauptbestandteilen Fibrinogen, Globulin und Albumin. Eine gewisse Anreicherung des Antihämophilie-Faktors VIII und eine gewisse Abtrennung des Fibrinogens, Globulins und Albumins können erzielt werden durch Wiederholung der Cohn-Fraktionierung mit Ethanol, hierbei soll jedoch die Temperatur nicht über 0°C steigen, da bei höheren Temperaturen eine Denaturierung des Proteins beobachtet wird.

Die Erfindung hat sich die Aufgabe gestellt, ein Verfahren zur Herstellung eines hochgereinigten Antihämophilie-Faktors (AHF oder Faktor VIII) zu entwickeln durch Abtrennung des Fibrinogens, Globulins und Albumins aus dem Kryoprezipitat.

Es wurde überraschenderweise gefunden, daß diese Aufgabe gelöst werden kann dadurch, daß

a) das aufgetaute Kryoprezipitat bei 10 bis 20°C in etwa dem doppelten Volumen Wasser, enthaltend 1 bis 3 U/ml Heparin-Natrium, suspendiert,

b) mit verdünnter Essigsäure auf pH 7,0 bis 8,0 eingestellt,

c) langsam, unter Rühren bei 10 bis 20°C mit etwa dem gleichen Volumen wässrigem Ethanol (7 bis 30% Gew/Vol), enthaltend 1 bis 3 U/ml Heparin-Natrium, versetzt,

d) mit verdünnter Essigsäure auf pH 6,8 bis 7,2 eingestellt,

e) unter Rühren langsam auf 10 bis 15°C abgekühlt,

f) der Niederschlag abgetrennt und

g) der Überstand in an sich bekannter Weise weiter aufgearbeitet wird.

Vorzugsweise erfolgt die Aufarbeitung der Stufe g) dadurch, daß Aluminiumhydroxid zugesetzt, dann abzentrifugiert und filtriert wird.

Um die Gefahr der Virusinfektion auszuschließen, wird vorzugsweise im Anschluß an die Stufen g) und h) eine Virusinaktivierung durchgeführt. Besonders bewährt hat sich die Virusinaktivierung mit Hilfe von Natrium-Cholat/TNBP.

Durch die erfindungsgemäßen Maßnahmen ist es erstmals möglich, in hohen Ausbeuten einen hochgereinigten Antihämophilie-Faktor herzustellen, der bezüglich der Reinheit allen bisher auf dem Markt befindlichen Produkten überlegen ist.

Zur Durchführung des erfindungsgemäßen Verfahrens wird handelsübliches Kryoprezipitat bei Raumtemperatur innerhalb von 3 bis 4 Stunden aufgetaut und in Stücke von ca. 1 bis 2 cm zerteilt.

Diese Stücke werden bei Temperaturen zwischen 10 und 20°C durch Rühren suspendiert in etwa dem doppelten Volumen Wasser, welches 1 bis 3 U/ml Heparin-Natrium enthält. Die Suspension wird mit verdünnter Essigsäure auf einen pH-Wert von mindestens 7,0, aber höchstens 8,0 eingestellt. Dieser Suspension wird jetzt langsam unter Rühren bei 10 bis 20°C etwa das gleiche Volumen wässriges Ethanol zugegeben. Der Ethanolgehalt sollte zwischen 7 und 30 Gew/Vol, vorzugsweise 10 bis 20 Gew/Vol, betragen. Auch diese Lösung enthält 1 bis 3 U/ml Heparin-Natrium. Jetzt wird mit verdünnter Essigsäure auf pH 6,8 bis 7,2 eingestellt und unter Rühren langsam auf 10 bis 15°C abgekühlt. Der jetzt verbliebene Niederschlag wird abgetrennt, beispielsweise durch Zentrifugieren, und der Überstand in an sich bekannter Weise weiter aufgearbeitet. Dieser Überstand enthält die Hauptmenge des Antihämophilie-Faktors und kaum noch Fibrinogen, Globulin und Albumin.

Die Aufarbeitung der Stufe g) erfolgt beispielsweise durch Zusatz von Aluminiumhydroxid, Abzentrifugieren und Filtrieren, wodurch insbesondere die als Verunreinigung enthaltenen Blutfaktoren VII, IX, X und Prothrombin entfernt werden. Nach dem Abzentrifugieren und Filtrieren erfolgt vorzugsweise eine Virusinaktivierung. Besonders bewährt hat sich die Virusinaktivierung mit Hilfe von Natrium-Cholat/TNBP. Das Natrium-Cholat/TNBP kann beispielsweise durch Ölextraktion wieder entfernt werden. Die wässrige Schicht wird beispielsweise durch ein Ultrafilter gereinigt und kann danach in üblicher Weise in Ampullen gefüllt und therapeutisch eingesetzt werden.

## Patentansprüche

1. Verfahren zur Herstellung eines hochgereinigten Antihämophilie-Faktors (AHF oder Faktor VIII) durch Abtrennung von Fibrinogen, Globulin und Albumin aus dem Kryoprezipitat, dadurch gekennzeichnet, daß

a) das aufgetaute Kryoprezipitat bei 10 bis 20°C in etwa dem doppelten Volumen Wasser, enthaltend 1 bis 3 U/ml Heparin-Natrium, suspendiert,

b) mit verdünnter Essigsäure auf pH 7,0 bis 8,0 eingestellt,

c) langsam, unter Rühren bei 10 bis 20°C mit etwa dem gleichen Volumen wässrigem Ethanol (7 bis 30% Gew/Vol), enthaltend 1 bis 3 U/ml Heparin-Natrium, versetzt,

d) mit verdünnter Essigsäure auf pH 6,8 bis 7,2 eingestellt,

e) unter Rühren langsam auf 10 bis 15°C abgekühlt,

f) der Niederschlag abgetrennt und

g) der Überstand in an sich bekannter Weise weiter aufgearbeitet wird.

**2.** Verfahren gemäß Anspruch 1, dadurch gekennzeichnet, daß die Aufarbeitung der Stufe g) erfolgt durch

h) Zusatz von Aluminiumhydroxid, Abzentrifugieren und Filtrieren.

**3.** Verfahren gemäß Anspruch 1 oder 2, dadurch gekennzeichnet, daß im Anschluß an die Stufen g) und h) eine Virusinaktivierung erfolgt mit Hilfe von Natrium-Cholat/TNBP.

**Claims**

**1.** A process for preparing a highly purified antihemophilia factor (AHF or factor VIII) by removing fibrinogen, globulin, and albumin from the cryoprecipitate, characterized in that

a) the thawed cryoprecipitate is suspended at 10 to 20°C in about the double volume of water containing from 1 to 3 U/ml of heparin sodium;

b) adjusted to pH 7.0 to 8.0 using dilute acetic acid;

c) slowly treated at 10 to 20°C with about the same volume of aqueous ethanol (7 to 30% wt./vol.) containing from 1 to 3 U/ml of heparin sodium;

d) adjusted to pH 6.8 to 7.2 using dilute acetic acid;

e) slowly cooled to 10 to 15°C with stirring;

f) the precipitate is removed; and

g) the supernatant is processed in a per se known manner.

**2.** Process according to claim 1, characterized in that the processing of step g) is accomplished by

h) addition of aluminum hydroxide, centrifugation, and filtration.

**3.** Process according to claim 1 or 2, characterized in that following steps g) and h), a virus inactiviation is accomplished using sodium cholate/TNBP.

**Revendications**

**1.** Procédé de production d'un facteur anti-hémophilique (AHF ou facteur VIII) par séparation du fibrinogène, de la globuline et de l'albumine du cryo-précipité, caractérisé en ce que :

a) le cryo-précipité décongelé est mis en suspension à une température de 10 à 20°C dans à peu près le double de volume d'eau contenant de 1 à 3 U/ml d'héparine-sodium;

b) il est réglé à pH de 7,0 à 8,0 à l'aide d'acide acétique dilué;

c) il est mélangé lentement sous agitation à 10 à 20°C avec environ le même volume d'éthanol aqueux (7 à 30 % en poids/volume) contenant 1 à 3 U/ml d'héparine-sodium;

d) il est réglé à pH de 6,8 à 7,2 avec de l'acide acétique dilué;

e) il est refroidi lentement sous agitation à 10 à 15°C;

f) le précipité est séparé ; et

g) la phase surnageante est de manière connue en soi soumise à un autre traitement.

**2.** Procédé selon la revendication 1, caractérisé en ce que le traitement de la phase g) se fait par :

h) addition d'hydroxyde de sodium, séparation par centrifugation et filtration.

**3.** Procédé selon la revendication 1 ou 2, caractérisé en ce que, à la suite des phases g) et h), on procède à une inactivation virale à l'aide de cholate de sodium/TNBP.